# EUROPÄISCHE PATENTSCHRIFT

(11) **EP 1 613 571 B1**
(45) Veröffentlichungstag und Bekanntmachung des Hinweises auf die Patenterteilung: **09.12.2009**
(21) Anmeldenummer: 03782168.3
(22) Anmeldetag: 18.12.2003
(51) Int. Cl.: C07B 59/00, C07C 229/08, A61K 31/198, A61P 25/00

(54) **DEUTERIERTE CATECHOLAMINDERIVATE SOWIE DIESE VERBINDUNGEN ENTHALTENDE ARZNEIMITTEL**
DEUTERATED CATECHOLAMINE DERIVATIVES AND MEDICAMENTS COMPRISING SAID COMPOUNDS
DERIVES DE CATECHOLAMINES DEUTERES ET MEDICAMENTS CONTENANT LESDITS COMPOSES

(30) Priorität: 19.12.2002 DE 10261807
(43) Veröffentlichungstag der Anmeldung: 11.01.2006
(73) Patentinhaber: BDD Berolina Drug Development GmbH, 15366 Neuenhagen (DE)
(72) Erfinder: ALKEN, Rudolf-Giesbert, 16341 Zepernick (DE)
(74) Vertreter: Schubert, Klemens
(86) Internationale Anmeldenummer: PCT/DE2003/004203
(87) Internationale Veröffentlichungsnummer: WO 2004/056724

(56) Entgegenhaltungen:
- EP-A- 0 357 565
- DE-A- 2 049 115
- F. BINNS ET AL: "Deuteration and Tritiation of Aryl AldehydesStudies related to the chemistry of melanins. Part IX. Syntheses of Specifically Deuterated 3,4-Dihydroxyphenethylamines and (+-)-3,4-Dihydroxyphenylalanines" J. CHEM. SOC. C, 1970, Seiten 2049-51, XP009030719 in der Anmeldung erwähnt
- OGURA C ET AL: "Clinical effect of L-dopa on schizophrenia" DIALOG MEDLINE, 1976, XP002108867

## Beschreibung

Die Erfindung betrifft deuterierte Catecholaminderivate sowie diese Verbindungen enthaltende Arzneimittel.

Bekannte Vertreter der Catecholamine, wie das L-Dopa (Levodopa) sowie dessen Carbonsäureester, werden unter anderem zur Therapie von Morbus Parkinson und des Restless-Legs-Syndroms eingesetzt. Ein solches Arzneimittel, das Levodopa enthält ist beispielsweise Dopaflex^{®}. L-Dopa wirkt auf den Dopaminspiegel in den Nervenzellen des Gehirns. Anders als Dopamin selbst kann es die Blut-Hirn-Schranke passieren und wird im Gehirn zu Dopamin umgewandelt.

Weiterhin wird Levodopa in Arzneimitteln in Kombination mit aktiven Zusatzstoffen verabreicht. Es werden Kombinationen von Levodopa mit peripheren Decarboxylasehemmern, mit Hemmstoffen für das Enzym Catechol-O-Methyltransferase (COMT), mit Hemmstoffen für das Enzym Monoaminoxidase (MAO) und für die Dopamin-β-Hydroxylase verwendet.

In diesem Zusammenhang verwendete Decarboxylasehemmer sind beispielsweise D,L-Serin-2-(2,3,4-trihydroxybenzyl)hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid. Beispiele für Kombinationspräparate aus Levodopa und Decarboxylasehemmern sind unter anderem Madopar^{®} (Levodopa und Benserazid-Hydrochlorid) sowie Nacom^{®} (Levodopa und Carbidopa).

Beispiele für COMT-Hemmer sind Entacapon (Comtan^{®}) und Cabergolin und häufig verwendete MAO-Hemmer sind Selegilin-Hydrochlorid, Moclobemid und Tranylcypromin.

Als Hemmstoffe für die Dopamin-β-Hydroxylase werden Calcium-5-butylpicolinat und Calcium-5-pentylpicolinat beschrieben (DE 2 049 115).

Eine Aufgabe der vorliegenden Erfindung ist es, deuterierte Catecholaminderivate bereitzustellen, die gegenüber den bereits bekannten Verbindungen verbesserte pharmakokinetische und/oder pharmakodynamische Eigenschaften aufweisen, sowie Catecholaminderivate bereitzustellen, die zur Prophylaxe von Psychosen, unter anderem von Schizophrenie, eingesetzt werden können und zur Herstellung von Arzneimitteln zur Prophylaxe von Psychosen verwendet werden können.

F. Binns et al. beschreiben in "Studies related to the Chemistry of Melanins. Part IX. Syntheses of Specifically Deuteriated 3,4-Dihydroxyphenethylamines and (±)-3,4-Dihydroxyphenylalanines" J. Chem. Sec. (C), 1970, 1134-1138, die Synthese von in der β-Position dideuterierten oder in der 2,5 oder 6-postition monodeuterierten 3,4-dihydroxy-phenylalaninen. Die pharmakologische Wirkung dieser Verbindungen wird nicht offenbart.

EP-A-0 357 565 offenbart ein Herstellungsverfahren für das Antiparkinsonmittel Levodopa. Die Herstellung deuterierter Verbindungen wird nicht offenbart.

Ogura et al. beschreiben in "Clinical effect of L-dopa on schizophrenia" Curr. Ther. Res. Clin. Exp., 1976, 20 (3), 308-18, die therapeutische Verwendung von Levodopa bei Schizophrenie. Die Verwendung deuterierter Verbindungen wird nicht offenbart.

Überraschenderweise wurde gefunden, dass die erfindungsgemäßen deuterierten Catecholaminderivate wesentlich bessere pharmakokinetische und/oder pharmakodynamische Eigenschaften aufweisen, als die undeuterierten Verbindungen und dass sie außerdem zur Prophylaxe von Psychosen eingesetzt werden können und zur Herstellung von Arzneimitteln zur Prophylaxe von Psychosen verwendet werden können.

Erfindungsgemäß wird die Aufgabe also gelöst durch die Bereitstellung von Verbindungen der allgemeinen Formel I: 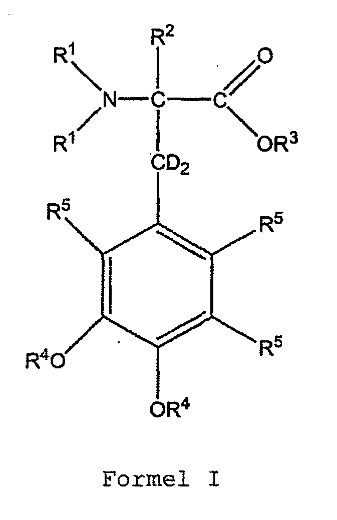 worin R¹ H oder D ist, R² H oder D bedeutet, R³ H, D, C₁-C₆-Alkyl oder C₅- bis C₆-Cycloalkyl, deuteriertes C₁- bis C₆-Alkyl oder deuteriertes C₅- bis C₆-Cycloalkyl ist, R⁴ H oder D bedeutet und R⁵ H oder D ist, mit Ausnahme von L-2-Amino-3,3-dideutero-3-(3,4-dihydroxyphenyl)-propionsaüre. Bevorzugt sind deuterierte Catecholaminderivate gemäß der allgemeinen Formel I, wobei R¹ H oder D ist, R² H oder D bedeutet, R³ H, D, C₁- bis C₆-Alkyl oder C₅- bis C₆-Cycloalkyl, deuteriertes C₁- bis C₆-Alkyl oder deuteriertes C₅- bis C₆-Cycloalkyl ist, R⁴ H oder D bedeutet und R⁵ D ist.

Insbesondere bevorzugt sind deuterierte Catecholaminderivate gemäß der allgemeinen Formel I, wobei R¹ H oder D ist, R² D bedeutet, R³ D, C₁- bis C₆-Alkyl oder C₅- bis C₆-Cycloalkyl, deuteriertes C₁- bis C₆₋Alkyl oder deuteriertes C₅- bis C₆-Cycloalkyl ist, R⁴ H oder D bedeutet und R⁵ D ist.

Weiterhin bevorzugt sind deuterierte Catecholaminderivate gemäß der allgemeinen Formel I, wobei R¹ H oder D ist, R² D bedeutet, R³ H, D, C₁- bis C₆-Alkyl oder C₅- bis C₆-Cycloalkyl, deuteriertes C₁- bis C₆-Alkyl oder deuteriertes C₅- bis C₆-Cycloalkyl ist, R⁴ H oder D bedeutet und R⁵ D ist.

Besonders vorteilhaft sind deuterierte Catecholaminderivate gemäß der allgemeinen Formel I,
wobei R¹ H oder D ist, R² D bedeutet, R³ C₁- bis C₆-Alkyl oder C₅- bis C₆-Cycloalkyl ist, R⁴ H oder D bedeutet und R⁵ D ist.

Weiterhin vorteilhaft sind deuterierte Catecholaminderivate gemäß der allgemeinen Formel I,
wobei R¹ H oder D ist, R² D bedeutet, R³ Methyl ist, R⁴ H oder D bedeutet und R⁵ D ist.

Insbesondere vorteilhaft sind deuterierte Catecholaminderivate gemäß der allgemeinen Formel I,
wobei R¹ H oder D ist, R² D bedeutet, R³ Ethyl ist, R⁴ H oder D bedeutet und R⁵ D ist.

Bevorzugt sind deuterierte Catecholaminderivate gemäß der allgemeinen Formel I, wobei R¹ H oder D ist, R² D bedeutet, R³ Perdeuteroethyl ist, R⁴ H oder D bedeutet und R⁵ D ist.

Weiterhin bevorzugt sind deuterierte Catecholaminderivate gemäß der allgemeinen Formel I, wobei R¹ H oder D ist, R² H oder D bedeutet, R³ Perdeuteroethyl ist, R⁴ H oder D bedeutet und R⁵ D ist.

Weiterhin bevorzugt sind deuterierte Catecholaminderivate gemäß der allgemeinen Formel I, wobei R¹ H oder D ist, R² H oder D bedeutet, R³ Perdeuteroethyl ist, R⁴ D bedeutet und R⁵ H oder D ist.

Besonders bevorzugt sind folgende deuterierte Catecholaminderivate gemäß der allgemeinen Formel I:
L-2-Amino-3,3-dideutero-3-(3,4-dihydroxyphenyl)-propionsäuremethylester,
L-2-Amino-3,3-dideutero-3-(3,4-dihydroxyphenyl)-propionsäureethylester,
L-2-Amino-3,3-dideutero-3-(3,4-dihydroxyphenyl)-propionsäurecyclohexylester,
L-2-Amino-3,3-dideutero-3-(3,4-dihydroxyphenyl)-propionsäure-perdeuteromethylester,
L-2-Amino-3,3-dideutero-3-(3,4-dihydroxyphenyl)-propionsäure-perdeuteroethylester,
L-2-Amino-3,3-dideutero-3-(3,4-dihydroxyphenyl)-propionsäure-perdeuterocyclohexylester,
L-2-Amino-2,3,3-trideutero-3-(3,4-dihydroxyphenyl)-propionsäure,
L-2-Amino-2,3,3-trideutero-3-(3,4-dihydroxyphenyl)-propionsäuremethylester,
L-2-Amino-2,3,3-trideutero-3-(3,4-dihydroxyphenyl)-propionsäureethylester,
L-2-Amino-2,3,3-trideutero-3-(3,4-dihydroxyphenyl)-propionsäurecyclohexylester,
L-2-Amino-2,3,3-trideutero-3-(3,4-dihydroxyphenyl)-propionsäure-perdeuteromethylester,
L-2-Amino-2,3,3-trideutero-3-(3,4-dihydroxyphenyl)-propionsäure-perdeuteroethylester,
L-2-Amino-2,3,3-trideutero-3-(3,4-dihydroxyphenyl)-propionsäure-perdeuterocyclohexylester,
L-2-Amino-2,3,3-trideutero-3-(2,3,6-trideutero-4,5-dihydroxyphenyl)propionsäure,
L-2-Amino-2,3,3-trideutero-3-(2,3,6-trideutero-4,5-dihydroxyphenyl)propionsäuremethylester,
L-2-Amino-2,3,3-trideutero-3-(2,3,6-trideutero-4,5-dihydroxyphenyl)propionsäureethylester,
L-2-Amino-2,3,3-trideutero-3-(2,3,6-trideutero-4,5-dihydroxyphenyl)propionsäurecyclohexylester,
L-2-Amino-2,3,3-trideutero-3-(2,3,6-trideutero-4,5-dihydroxyphenyl)propionsäure-perdeuteromethylester,
L-2-Amino-2,3,3-trideutero-3-(2,3,6-trideutero-4,5-dihydroxyphenyl)propionsäure-perdeuteroethylester,
L-2-Amino-2,3,3-trideutero-3-(2,3,6-trideutero-4,5-dihydroxyphenyl)propionsäure-perdeuterocyclohexylester,
L-2-Amino-2,3,3-trideutero-3-(2,3,6-trideutero-4,5-di-deuteroxyphenyl)propionsäure-perdeuterocyclohexylester,
L-2-Amino-3,3-dideutero-3-(4,5-dideuteroxyphenyl)-propionsäure-perdeuterocyclohexylester.

Eine weitere Ausführungsform der Erfindung ist die Verwendung der erfindungsgemäßen deuterierten Catecholaminderivate sowie deren physiologisch verträglicher Salze zur Herstellung von Arzneimitteln zur Behandlung von Dopaminmangelerkrankungen bzw. Erkrankungen die auf gestörtem Tyrosintransport oder gestörter Tyrosindecarboxylase beruhen wie Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie.

Bevorzugt ist hierbei die Verwendung der deuterierten Catecholaminderivate sowie deren physiologisch verträglicher Salze, in Kombination mit einem Enzymhemmer oder mehreren Enzymhemmern, zur Herstellung von Arzneimitteln zur Behandlung von Dopaminmangelerkrankungen bzw. Erkrankungen die auf gestörtem Tyrosintransport oder gestörter Tyrosindecarboxylase beruhen wie Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie.

Vorteilhaft ist es, wenn es sich bei dem Enzymhemmer bzw, den Enzymhemmern um Decarboxylasehemmer und/oder Catechol-O-Methyltransferase-Hemmer und/oder Monoaminoxidase-Hemmer und/oder β-Hydroxylase-Hemmer handelt.

Besonders vorteilhaft ist es, wenn der Decarboxylasehemmer ausgewählt wird aus der Gruppe, bestehend aus D,L-Serin-2-(2,3,4-trihydroxybenzyl)-hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

Insbesondere vorteilhaft ist es außerdem, wenn der Catechol-O-Methyltransferase-Hemmer ausgewählt wird aus Entacapon und Cabergolin sowie deren physiologisch verträglicher Salze.

Bevorzugt ist es auch, wenn der Monoaminoxidase-Hemmer ausgewählt wird aus der Gruppe, bestehend aus Selegilin, Moclobemid und Tranylcypromin sowie deren physiologisch verträglicher Salze.

Besonders bevorzugt ist es weiterhin, wenn der β-Hydroxylase-Hemmer ausgewählt wird aus Calcium-5-butylpicolinat und Calcium-5-pentylpicolinat sowie deren physiologisch verträglicher Salze.

Ein weiterer Gegenstand der Erfindung ist die Verwendung der erfindungsgemäßen deuterierten Catecholaminderivate sowie deren physiologisch verträglicher Salze zur Herstellung von Arzneimitteln zur Behandlung von Dopaminmangelerkrankungen bzw. Erkrankungen die auf gestörtem Tyrosintransport oder gestörter Tyrosindecarboxylase beruhen wie Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie.

Eine anderer Gegenstand der vorliegenden Erfindung ist eine pharmazeutische Zusammensetzung, welche die erfindungsgemäßen deuterierten Catecholamine sowie deren physiologisch verträgliche Salze zur Behandlung von Dopaminmangelerkrankungen bzw. Erkrankungen die auf gestörtem Tyrosintransport oder gestörter Tyrosindecarboxylase beruhen wie Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie, neben pharmazeutisch verträglichen Hilfs- und Zusatzstoffen, enthält.

Besonders vorteilhaft ist hierbei eine pharmazeutische Zusammensetzung, welche die erfindungsgemäßen deuterierten Catecholamine sowie deren physiologisch verträgliche Salze zur Behandlung von Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie, sowie einen oder mehrere Enzymhemmer, neben pharmazeutisch verträglichen Hilfs- und Zusatzstoffen, enthält.

Insbesondere bevorzugt ist eine pharmazeutische Zusammensetzung, bei der es sich bei dem Enzymhemmer bzw. den Enzymhemmern um Decarboxylasehemmer und/oder Catechol-O-Methyltransferase-Hemmer und/oder Monoaminoxidase-Hemmer und/oder β-Hydroxylase-Hemmer handelt.

Weiterhin bevorzugt ist eine pharmazeutische Zusammensetzung bei welcher der Decarboxylasehemmer ausgewählt wird aus der Gruppe, bestehend aus D,L-Serin-2-(2,3,4-trihydroxybenzyl)hydrazid (Benserazid), (-)-L-α-Hydrazino-3,9-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

Besonders vorteilhaft ist eine pharmazeutische Zusammensetzung, bei welcher der Catechol-O-Methyltransferase-Hemmer ausgewählt wird aus Entacapon und Cabergolin sowie deren physiologisch verträglicher Salze.

Weiterhin vorteilhaft ist eine pharmazeutische Zusammensetzung, bei welcher der Monoaminoxidase-Hemmer ausgewählt wird aus der Gruppe, bestehend aus Selegilin, Moclobemid und Tranylcypromin sowie deren physiologisch verträglicher Salze.

Außerdem bevorzugt ist eine pharmazeutische Zusammensetzung, bei welcher der β-Hydroxylase-Hemmer ausgewählt wird aus Calcium-5-butylpicolinat und Calcium-5-pentylpicolinat sowie deren physiologisch verträglicher Salze.

Ein weiterer Gegenstand der Erfindung ist die Verwendung der erfindungsgemäßen deuterierten Catecholaminderivate sowie deren physiologisch verträglicher Salze zur Herstellung von Arzneimitteln zum Einsatz bei der Prophylaxe von Psychosen, insbesondere bei prädisponierten Patienten, zur Prophylaxe eines Rückfalls und insbesondere auch zur Behandlung von akuten Psychosen, zum Beispiel mit Negativsymptomatik.

Besonders bevorzugt ist hierbei die Verwendung der erfindungsgemäßen deuterierten Catecholaminderivate sowie deren physiologisch verträglicher Salze, in Kombination mit einem oder mehreren Enzymhemmer, zur Herstellung von Arzneimitteln zum Einsatz bei der Prophylaxe von Psychosen und zum Einsatz bei akuten Psychosen, vorzugsweise Psychosen mit Negativsymptomatik.

Weiterhin bevorzugt ist die Verwendung der erfindungsgemäßen deuterierten Catecholaminderivate sowie deren physiologisch verträglicher Salze, wenn es sich bei dem Enzymhemmer bzw. den Enzymhemmern um Decarboxylasehemmer und/oder Catechol-O-Methyltransferase-Hemmer und/oder Monoaminoxidase-Hemmer und/oder β-Hydroxylase-Hemmer handelt.

Insbesondere bevorzugt ist die Verwendung der erfindungsgemäßen deuterierten Catecholaminderivate sowie deren physiologisch verträglicher Salze, wenn der Decarboxylasehemmer ausgewählt wird aus der Gruppe, bestehend aus D,L-Serin-2-(2,3,4-trihydroxybenzyl)-hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

Vorteilhaft ist die Verwendung der erfindungsgemäßen deuterierten Catecholaminderivate sowie deren physiologisch verträglicher Salze, wenn der Catechol-O-Methyltransferase-Hemmer ausgewählt wird aus Entacapon und Cabergolin sowie deren physiologisch verträglicher Salze.

Weiterhin vorteilhaft ist hierbei die Verwendung der erfindungsgemäßen deuterierten Catecholaminderivate sowie deren physiologisch verträglicher Salze, wenn der Monoaminoxidase-Hemmer ausgewählt wird aus der Gruppe, bestehend aus Selegilin, Moclobemid und Tranylcypromin sowie deren physiologisch verträglicher Salze.

Besonders vorteilhaft ist die Verwendung der erfindungsgemäßen deuterierten Catecholaminderivate sowie deren physiologisch verträglicher Salze, wenn der β-Hydroxylase-Hemmer ausgewählt wird aus Calcium-5-butylpicolinat und Calcium-5-pentylpicolinat sowie deren physiologisch verträglicher Salze.

Ein weiterer Gegenstand der Erfindung ist die Verwendung der erfindungsgemäßen deuterierten Catecholaminderivate sowie deren physiologisch verträglicher Salze zur Herstellung von Arzneimitteln zum Einsatz bei der Prophylaxe von Psychosen.

Ein noch weiterer Gegenstand der Erfindung ist eine pharmazeutische Zusammensetzung, welche die erfindungsgemäßen deuterierten Catecholamine sowie deren physiologisch verträgliche Salze zum Einsatz bei der Prophylaxe von Psychosen und zur Behandlung von akuten Psychose, neben pharmazeutisch verträglichen Hilfs- und Zusatzstoffen, enthält.

Besonders vorteilhaft ist hierbei eine pharmazeutische Zusammensetzung, welche die erfindungsgemäßen deuterierten Catecholamine sowie deren physiologisch verträgliche Salze zur Prophylaxe von Psychosen und zur Therapie von akuten Psychosen, sowie einen oder mehrere Enzymhemmer, neben pharmazeutisch verträglichen Hilfs- und Zusatzstoffen, enthält.

Insbesondere bevorzugt ist eine pharmazeutische Zusammensetzung, bei welcher es sich bei dem Enzymhemmer bzw. den Enzymhemmern um Decarboxylasehemmer und/oder Catechol-O-Methyltransferase-Hemmer und/oder Monoaminoxidase-Hemmer und/oder β-Hydroxylase-Hemmer handelt.

Weiterhin vorteilhaft ist eine pharmazeutische Zusammensetzung, bei welcher der Decarboxylasehemmer ausgewählt wird aus der Gruppe, bestehend aus D,L-Serin-2-(2,3,4-trihydroxybenzyl)hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

Besonders vorteilhaft ist eine pharmazeutische Zusammensetzung, bei welcher der Catechol-O-Methyltransferase-Hemmer ausgewählt wird aus Entacapon und Cabergolin sowie deren physiologisch verträglicher Salze.

Insbesondere vorteilhaft ist eine pharmazeutische Zusammensetzung, bei welcher der Monoaminoxidase-Hemmer ausgewählt wird aus der Gruppe, bestehend aus Selegilin, Moclobemid und Tranylcypromin sowie deren physiologisch verträglicher Salze.

Besonders bevorzugt ist eine pharmazeutische Zusammensetzung, bei welcher der β-Hydroxylase-Hemmer ausgewählt wird aus Calcium-5-butylpicolinat und Calcium-5-pentylpicolinat sowie deren physiologisch verträglicher Salze.

Die Herstellung der erfindungsgemäßen L-2-Amino-3,3-dideutero-3-(3,4-dihydroxyphenyl)propionsäure erfolgt in Anlehnung an Binns et al., J. Chem. Soc. (C), 1970, Seiten 1134-1138, wo unter anderem die Herstellung von racemischer 2-Amino-3,3-dideutero-3-(3,4-dihydroxyphenyl)propionsäure beschrieben wird. Ausgehend von 3,4-Dimethoxybenzoesäureethylester wird Dideutero-(3,4-dimethoxyphenyl)methan durch die Umsetzung mit Lithiumaluminiumdeuterid hergestellt. Daraus wird durch Reaktion mit Thionylchlorid 4-(Chlordideuteromethyl)-1,2-dimethoxybenzol erzeugt, das mit dem Natriumsalz von Acetamidomalonsäurediethylester zu deuteriertem 3,4-Dimethoxybenzylacetamidomalonsäurediethylester umgesetzt, der durch die Behandlung mit ethanolischer Kaliumhydroxidlösung in D,L-2-Acetylamino-3,3-dideutero-3-(3,4-dimethoxyphenyl)propionsäure umgewandelt wird. Die erfindungsgemäße Aufgabe, das L-Enantiomere der in β,β-Position dideuterierten Aminosäure herzustellen konnte dadurch gelöst werden, dass an dieser Stelle eine Racematspaltung analog zu dem in der Patentschrift CH 59098 offenbarten Verfahren vorgenommen wurde. Es wurde gefunden, dass auch L-2-Acetylamino-3,3-dideutero-3-(3,4-dimethoxyphenyl)propionsäure sehr gut durch Kristallbildung mit (R)-(+)-1-Phenylethylamin aus der Lösung isoliert werden kann. Aus der L-2-Acetylamino-3,3-dideutero-3-(3,4-dimethoxyphenyl)propionsäure wurde dann durch eine schonende Etherspaltung, analog zu Jung et al., J. Org. Chem., Vol. 42, Nr. 23, 1977, S. 3761-3764, die erfindungsgemäße L-2-Amino-3,3-dideutero-3-(3,4-dihydroxyphenyl)propionsäure erhalten. Aus dieser Aminosäure wurden anschließend bei niedriger Temperatur durch Umsetzung mit Thionylchlorid und deuteriertem oder undeuteriertem Alkohol die erfindungsgemäßen Ester der in β-Position dideuterierten Verbindung hergestellt.

Von besonderem Vorteil ist hierbei, dass aus der Mutterlauge der Racematspaltung die verbliebene D-2-Acetylamino-3,3-dideutero-3-(3,4-dimethoxyphenyl)-propionsäure isoliert werden konnte, die nach der Etherspaltung als Edukt für die Herstellung weiterer erfindungsgemäßer Verbindungen verwendet werden konnte.

Weiterhin dient L-2-Amino-3,3-dideutero-3-(3,4-dihydroxyphenyl)propionsäure als Edukt für die zusätzliche Deuterierung im Phenylring der Aminosäure, indem die Verbindung analog zu Vining et al., Journal of Labelled Compounds and Radiopharmaceuticals, Vol. XVIII, Nr. 11, 1981, S. 1683-1692, bei 190 °C im Autoklav mit D₂O zur Reaktion gebracht wird. Die erhaltene L-2-Amino-3,3-dideutero-3-(2,3,6-trideutero-4,5-dihydroxyphenyl)-propionsäure wurde dann, wie oben beschrieben, in die erfindungsgemäßen Ester überführt. In Anlehnung an EP 610595 wurde bei der Esterherstellung bzw. -isolierung durch Zugabe von Antioxidationsmittel die Stabilität der gewonnen Ester erhöht.

Die durch die oben beschriebene Racematspaltung erhaltene D-2-Acetylamino-3,3-dideutero-3-(3,4-dimethoxyphenyl)-propionsäure wurde analog der L-Verbindung in die Dihydroxyaminosäure überführt und dann verwendet, um die in α-Position deuterierten, erfindungsgemäßen Verbindungen zu erzeugen, indem analog zu Chen et al., Biotechnology Letters, Vol. 14, Nr. 4, 1992, S. 269-274 eine Racemisierung mit gleichzeitiger Deuterierung erfolgte. Hierzu wurde D-2-Acetylamino-3,3-dideutero-3-(3,4-dihydroxyphenyl)propionsäure mit Benzaldehyd in deuterierter Essigsäure umgesetzt. Die als Racemat vorliegenden, in α-Position deuterierten D- und L-2-Acetylamino-3,3-dideutero-3-(3,4-dihydroxyphenyl)-propionsäuren wurden zu den entsprechenden Methylestern umgesetzt und mittels Alcalase getrennt, indem enzymatisch der L-2-Acetylamino-3,3-dideutero-3-(3,4-dihydroxyphenyl)propionsäuremethylester zur Carbonsäure hydrolysiert wurde und der Methylester der D-2-Acetylamino-3,3-dideutero-3-(3,4-dihydroxyphenyl)-propionsäure unumgesetzt verblieb. Die Trennung der Verbindungen erfolgte mittels HPLC.

Die isolierte L-2-Acetylamino-2,3,3-trideutero-3-(3,4-dihydroxyphenyl)propionsäure wurde in die erfindungsgemäßen Ester überführt bzw. entsprechend des oben bereits erläuterten Verfahrens zusätzlich am Phenylring deuteriert um die erfindungsgemäße L-2-Amino-2,3,3-trideutero-3-(2,3,6-trideutero-4,5-dihydroxyphenyl)propionsäure zu erhalten, die wiederum in die erfindungsgemäßen Ester überführt wurde.
Der H/D-Austausch an den phenolischen OH-Gruppen und an der Aminogruppe wurde durch mehrmaliges Umkristallisieren aus D₂O durchgeführt.

Zur Herstellung der physiologisch verträglichen Salze der erfindungsgemäßen deuterierten Catecholaminderivate können übliche, physiologisch verträgliche anorganische und organische Säuren wie Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure verwendet werden. Weitere verwendbare Säuren sind beispielweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.
Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wässrige Lösungen von Säureadditionssalzen der erfindungsgemäß verwendeten Verbindungen in einer wässrigen Säurelösung hergestellt werden.

Die Säureadditionssalze der erfindungsgemäßen Verbindungen können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, buccalen, sublingualen, nasalen, rektalen, subcutanen, intravenösen oder intramuskulären Applikation sowie zur Inhalation, die neben üblichen Träger- und Verdünnungsmitteln eine Verbindung der allgemeinen Formel I oder deren Säureadditionssalz als Wirkstoff enthalten.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Lutschtabletten, Filmtabletten, Dragees, Kapseln, Pillen, Pulver, Lösungen, Aerosole oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt. Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees, auch für kontrolliert oder verzögert freisetzende Zubereitungsformen, durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäß verwendeten Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln wie Neutralfetten oder Polyäthylenglykol bzw. deren Derivaten herstellen.

Die Herstellung der erfindungsgemäßen pharmazeutischen Zubereitungen ist an sich bekannt und in den dem Fachmann bekannten Handbüchern beschrieben, beispielsweise Hager's Handbuch (5.) 2, 622-1045; List et al., Arzneiformenlehre, Stuttgart: Wiss. Verlagsges. 1985; Sucker et al., Pharmazeutische Technologie, Stuttgart: Thieme 1991; Ullmann's Enzyklopädie (5.) A 19, 241-271; Voigt, Pharmazeutische Technologie, Berlin: Ullstein Mosby 1995.

Die folgenden Beispiele erläutern die Erfindung:

### Beispiel 1

### Herstellung von L-2-Acetylamino-3,3-dideutero-3-(3,4-dimethoxy-phenyl)-propionsäure

Analog zum Verfahren für die undeuterierte Verbindung werden zu 3,85 g D,L-2-Acetylamino-3,3-dideutero-3-(3,4-dimethoxyphenyl)propionsäure 50 ml Aceton hinzugefügt und die Lösung wird erwärmt. Zu dieser warmen Lösung werden 0,865 g (R)-(+)-1-Phenylethylamin, gelöst in 5 ml Aceton, hinzugefügt. Durch Zugabe von wenig Methanol wird ausgefallenes Salz wieder in Lösung gebracht. Das Methanol wird durch mehrmaliges Einengen des Reaktionsansatzes entfernt und das Volumen der Lösung durch Zugabe von Aceton auf 50 ml aufgefüllt. Zur Kristallisation des sich aus L-2-Acetylamino-3,3-dideutero-3-(3,4-dimethoxyphenyl)propionsäure und (R)-(+)-1-Phenylethylamin gebildeten Salzes wird der Reaktionsansatz mit einem Glasstab gerieben und nach einsetzender Kristallisation für 12 Stunden bei Raumtemperatur stehen gelassen. Die gebildeten Kristalle werden abgetrennt, mit kaltem Aceton und Diethylether gewaschen und getrocknet.
Es werden 2,6 g des Salzes isoliert.
Das Lösemittel wird von der verbliebenen Mutterlauge abdestilliert und der Rückstand aus D-2-Acetylamino-3,3-dideutero-3-(3,4-dimethoxyphenyl)propionsäure wird bis zur weiteren Verarbeitung aufbewahrt.
Ausbeute: 93% Schmelzpunkt: 185-187 °C
[α]_{D}²⁵= +56,4° (c = 1 in Methanol)

Das Salz wird ohne weitere Reinigung weiterverarbeitet, indem 2,5 g in 15 ml einer 5%-igen Natriumhydroxidlösung gelöst werden. Das freigesetzte (R)-(+)-1-Phenylethylamin wird aus der Lösung durch Extraktion mit Petrolether entfernt. Nach dem Ansäuern der wässrigen Phase mit Salzsäure wird eine gesättigte Natriumchloridlösung hinzugefügt und die Lösung mit Essigsäureethlester extrahiert. Die organische Phase wird getrocknet und das Lösemittel entfernt. Der Rückstand kristallisiert über Nacht aus und man erhält L-2-Acetylamino-3,3-dideutero-3-(3,4-dimethoxyphenyl)propionsäure. Man erhält 1,48 g Produkt.
Ausbeute: 86%
Schmelzpunkt: 135-137 °C
[α]_{D}²⁵ = +45,5° (c = 1 in Methanol)
berechnet:
C: 57,98 % H: 7,11 % N: 5,20 %
gefunden:
C: 57,89 % H: 7,19 % N: 5,30 %
¹H-NMR (400 MHz, d6-DMSO): δ 6,48 (s, 1H); 6,60 (s, 1H); 6,54 (s, 1H); 7,8 (s, 1H); 4,60 (s, 1H) ; 3,70 (s, 6H); 2,20 (s, 3H).

### Beispiel 2

### Herstellung von L-2-Amino-3,3-dideutero-3-(3,4-dihydroxyphenyl)-propionsäure

1,35 g L-2-Acetylamino-3,3-dideutero-3-(3,4-dimethoxyphenyl)propionsäure werden in 17 ml Chloroform gelöst und anschließend mit 26,3 ml Iodtrimethlysilan versetzt. Der Reaktionsansatz wird auf 60 °C erhitzt und der Ablauf der Reaktion mittels NMR verfolgt. Nach 30 Stunden ist die Reaktion beendet, der Ansatz wird filtriert und zum Filtrat werden 15 ml Methanol hinzugefügt. Nach 30 Stunden wird das Lösemittel entfernt und man isoliert 0,96 g Produkt.
Ausbeute: 96%
Schmelzpunk: 287-290 °C (Zers.)
[α]_{D}²⁵ = -11,7°(c = 5,27 in 1 M HCl)
berechnet:
C: 54,27 % H: 6,58 % N: 7,03 %
gefunden:
C: 54,10 % H: 6,60 % N: 7,11 %
¹H-NMR (400 MHz, d6-DMSO): δ 6,49 (s, 1H) ; 6,59 (s, 1H); 6,54 (s, 1H); 7,8 (s, 1H); 4,28 (s, 1H).

### Beispiel 3

### D-2-Amino-3,3-dideutero-3-(3,4-dihydroxyphenyl)-propionsäure

Die in Beispiel 1 gewonnene D-2-Acetylamino-3,3-dideutero-3-(3,4-dimethoxyphenyl)propionsäure wird analog zu Beispiel 2 in die D-2-Amino-3,3-dideutero-3-(3,4-dihydroxyphenyl)propionsäure überführt. Aus 1,2 g der Ausgangsverbindung werden 0,82 g der deuterierten Dihydroxyaminosäure isoliert.
Ausbeute: 92%
Schmelzpunk: 287-290 °C (Zers.)
[α]_{D}²⁵ = +11,5°(c = 5,27 in 1 M HCl)
berechnet:
C: 54,27 % H: 6,58 % N: 7,03 %
gefunden:
C: 54,31 % H: 6,55 % N: 7,10 %
¹³C-NMR (200 MHz, d6-DMSO): δ 41,0 (quint); 62,50 (s); 116,20 (s); 117,30 (s); 121,70 (s); 133,80 (s); 141,40 (s); 144, 40 (s); 176,40 (s).

### Beispiel 4

### Herstellung von D,L-2-Amino-2,3,3-trideutero-3-(3,4-dihydroxyphenyl)propionsäure

1,99 g D-2-Acetylamino-3,3-dideutero-3-(3,4-dihydroxyphenyl)propionsäure werden mit 50 ml einfach deuterierter Essigsäure (CH₃COOD) versetzt und es werden 0,2 ml Benzaldehyd hinzugefügt. Der Reaktionsansatz wird mit Stickstoff gespült und anschließend für eine Stunde zum Rückfluss erhitzt. Nach beendeter Reaktionszeit wird das Lösemittel entfernt und der Rückstand mit 20 ml Ethanol versetzt. Der ausgefallene Feststoff wird abfiltriert und man isoliert 1,74 g D,L-2-Amino-2,3,3-trideutero-3-(3,4-dihydroxyphenyl)propionsäure.
Ausbeute: 87%
Schmelzpunkt: 287-290 °C (Zers.)
berechnet:
C: 53,99 % H: 7,05 % N: 7,00 %
gefunden:
C: 53,90 % H: 7,12 % N: 7,04 %
¹H-NMR (400 MHz, d6-DMSO): δ 6,47 (s, 1H); 6,59 (s, 1H); 6,52 (s, 1H); 7,8 (s, 1H).

### Beispiel 5

### Herstellung von D,L-2-Amino-2,3,3-trideutero-3-(3,4-dihydroxyphenyl)propionsäuremethylester

2 g D,L-2-Amino-2,3,3-trideutero-3-(3,4-dihydroxyphenyl)propionsäure in 30 ml Methanol werden auf -10 °C gekühlt und tropfenweise mit 1 ml Thionylchlorid versetzt. Der Reaktionsansatz wird dann für 15 Stunden auf 40 °C erwärmt. Der Reaktionsansatz wird im Vakuum von flüchtigen Substanzen befreit und es werden 10 ml Wasser und 15 ml einer Lösung aus 0,8 g Natriumhydrogencarbonat, 1 g Natriumsulfat und 1 mg Ascorbinsäure hinzugefügt. Durch Zugabe einer verdünnten Natriumhydroxidlösung wird der pH-Wert der Lösung auf einen Wert von 7 eingestellt. Das Produkt wird durch Extraktion mit sauerstofffreiem Essigsäureethylester, der 0,01% 2,6-Di-tert-butyl-4-methoxyphenol enthält, in die organische Phase überführt. Die organische Phase wird getrocknet und anschließend das Lösemittel abdestilliert. Dem Rückstand werden 50 ml sauerstofffreier Diethylether hinzugesetzt und nach Stehenlassen über Nacht scheidet sich der D,L-2-Amino-2,3,3-trideutero-3-(3,4-dihydroxyphenyl)propionsäure-methylester ab. Nach Umkristallisation aus einem sauerstofffreiem, mit 2,6-Di-tert-butyl-4-methoxyphenol versetzten, Methanol/ Diethylethergemisch werden 1,8 g Produkt isoliert.
Ausbeute: 85%
berechnet:
C: 56,06 % H: 7,53 % N: 6,54 %
gefunden:
C: 56,20 % H: 7,48 % N: 6,55 %
¹H-NMR (400 MHz, d6-DMSO): δ 6,48 (s, 1H); 6,59 (s, 1H); 6,54 (s, 1H); 7,8 (s, 1H); 3,80 (s, 3H).

### Beispiel 6

### L-2-Amino-2,3,3-trideutero-3-(3,4-dihydroxyphenyl)-propionsäure

1,07 g D,L-2-Amino-2,3,3-trideutero-3-(3,4-dihydroxyphenyl)-propionsäuremethylester werden in 30 ml einer 0,2-molaren Natriumbicarbonatlösung (pH 8,2) gelöst. Es werden 200 µl Alcalase hinzugesetzt und der pH-Wert der Lösung wird mittels eines Carbonat-Bicarbonatpuffers auf diesem Wert gehalten. Der Reaktionsverlauf wird mittels HPLC kontrolliert und die Reaktion durch Zugabe von Salzsäure beendet, als sich die Konzentration des Esters auf die Hälfte reduziert hatte. Die in der Lösung enthaltene trideuterierte Aminosäure wird von dem trideuterierten Methylester chromatographisch unter Verwendung des Laufmittelgemischs Acetonitril/ 0,1% wässrige Trifluoressigsäure (15:85) abgetrennt und es werden 1,04 g L-2-Amino-2,3,3-trideutero-3-(3,4-dihydroxyphenyl)propionsäure isoliert.
Ausbeute: 97%
Schmelzpunkt: 287-290 °C (Zers.)
[α]_{D}²⁵ = -11,6° (c = 5,27 in 1 M HCl)
C: 53,99 % H: 7,05 % N: 7,00 %
gefunden:
C: 53,83 % H: 7,12 % N: 6,91 %
¹³C-NMR (200 MHz, d6-DMSO): δ 41,0 (quint); 62,40 (trip.); 116,20 (s); 117,30 (s); 121,70 (s); 133,80 (s); 141,40 (s); 144,40 (s); 176,40 (s).

### Beispiel 7

### Herstellung von L-2-Amino-2,3,3-trideutero-3-(2,3,6-trideutero-4,5-dihydroxyphenyl)propionsäure

In einem Autoklav werden 0,2 g L-2-Amino-2,3,3-trideutero-3-(3,4-dihydroxyphenyl)propionsäure mit 10 ml D₂O versetzt. Der Autoklav wird evakuiert und für 24 Stunden auf eine Temperatur von 190 °C erhitzt. Nach Beendigung der Reaktion wird das Lösemittel entfernt, der Rückstand mit Essigsäureethylester versetzt und das Lösemittel im Vakuum abdestilliert. Der Rückstand wird mit kaltem Aceton gewaschen und man isoliert 0,17 g Produkt.
Ausbeute: 84%
Schmelzpunkt: 287-290 °C (Zers.)
[α]_{D}²⁵ = -11,5° (c = 5,27 in 1 M HCl)
C: 53,19 % H: 8,43 % N: 6,89 %
gefunden:
C: 53,30 % H: 8,31 % N: 7,00 %
¹³C-NMR (200 MHz, d6-DMSO): δ 41,0 (quint); 62,40 (t); 116,30 (t); 117,20 (t); 121,70 (t); 133,80 (s); 141,30 (s); 144,40 (s); 176,40 (s).

## Patentansprüche

1. Deuterierte Catecholaminderivate der allgemeinen Formel I, 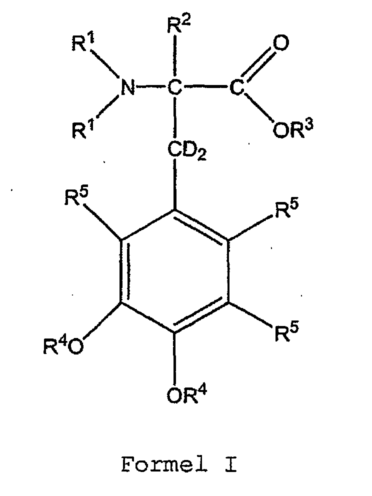 wobei
R¹ H oder D ist, R² H oder D bedeutet, R³ H, D, C₁-C₆-Alkyl oder C₅- bis C₆-Cycloalkyl, deuteriertes C₁- bis C₆-Alkyl oder C₅- bis C₆-Cycloalkyl ist, R⁴ H oder D bedeutet und R⁵ H oder D ist, mit Ausnahme von L-2-Amino-3,3-dideutero-3-(3,4-dihydroxyphenyl)-propionsäure.

2. Deuterierte Catecholaminderivate gemäß Anspruch 1, wobei R¹ H oder D ist, R² H oder D bedeutet, R³ H, D, C₁- bis C₆-Alkyl oder C₅- bis C₆-Cycloalkyl, deuteriertes C₁- bis C₆-Alkyl oder deuteriertes C₅-bis C₆-Cycloalkyl ist, R⁴ H oder D bedeutet und R⁵ D ist.

3. Deuterierte Catecholaminderivate gemäß Anspruch 1, wobei R¹ H oder D ist, R² D bedeutet, R³ D, C₁- bis C₆-Alkyl oder C₅- bis C₆-Cycloalkyl, deuteriertes C₁-bis C₆-Alkyl oder deuteriertes C₅- bis C₆-Cycloalkyl ist, R⁴ H oder D bedeutet und R⁵ D ist.

4. Deuterierte Catecholaminderivate gemäß Anspruch 1, wobei R¹ H oder D ist, R² D bedeutet, R³ H, D, C₁- bis C₆-Alkyl oder C₅- bis C₆-Cycloalkyl, deuteriertes C₁-bis C₆-Alkyl oder deuteriertes C₅- bis C₆-Cycloalkyl ist, R⁴ H oder D bedeutet und R⁵ D ist.

5. Deuterierte Catecholaminderivate gemäß der allgemeinen Formel I, wobei R¹ H oder D ist, R² D bedeutet, R³ C₁- bis C₆-Alkyl oder C₅- bis C₆-Cycloalkyl ist, R⁴ H oder D bedeutet und R⁵ D ist.

6. Deuterierte Catecholaminderivate gemäß Anspruch 1, wobei R¹ H oder D ist, R² D bedeutet, R³ Methyl ist, R⁴ H oder D bedeutet und R⁵ D ist.

7. Deuterierte Catecholaminderivate gemäß Anspruch 1, wobei R¹ H oder D ist, R² D bedeutet, R³ Ethyl ist, R⁴ H oder D bedeutet und R⁵ D ist.

8. Deuterierte Catecholaminderivate gemäß Anspruch 1, wobei R¹ H oder D ist, R² D bedeutet, R³ Perdeuteroethyl ist, R⁴ H oder D bedeutet und R⁵ D ist.

9. Deuterierte Catecholaminderivate gemäß Anspruch 1, wobei R¹ H oder D ist, R² H oder D bedeutet, R³ Perdeuteroethyl ist, R⁴ H oder D bedeutet und R⁵ D ist.

10. Deuterierte Catecholaminderivate gemäß Anspruch 1, wobei R¹ H oder D ist, R² H oder D bedeutet, R³ Perdeuteroethyl ist, R⁴ D bedeutet und R⁵ H oder D ist.

11. Deuterierte Catecholaminderivate gemäß Anspruch 1, nämlich
L-2-Amino-3,3-dideutero-3-(3,4-dihydroxyphenyl)-propionsäuremethylester,
L-2-Amino-3,3-dideutero-3-(3,4-dihydroxyphenyl)-propionsäureethylester,
L-2-Amino-3,3-dideutero-3-(3,4-dihydroxyphenyl)-propionsäurecyclohexylester,
L-2-Amino-3,3-dideutero-3-(3,4-dihydroxyphenyl)-propionsäure-perdeuteromethylester,
L-2-Amino-3,3-dideutero-3-(3,4-dihydroxyphenyl)-propionsäure-perdeuteroethylester,
L-2-Amino-3,3-dideutero-3-(3,4-dihydroxyphenyl)-propionsäure-perdeuterocyclohexylester,
L-2-Amino-2,3,3-trideutero-3-(3,4-dihydroxyphenyl)-propionsäure,
L-2-Amino-2,3,3-trideutero-3-(3,4-dihydroxyphenyl)-propionsäuremethylester,
L-2-Amino-2,3,3-trideutero-3-(3,4-dihydroxyphenyl)-propionsäureethylester,
L-2-Amino-2,3,3-trideutero-3-(3,4-dihydroxyphenyl)-propionsäurecyclohexylester,
L-2-Amino-2,3,3-trideutero-3-(3,4-dihydroxyphenyl)-propionsäure-perdeuteromethylester,
L-2-Amino-2,3,3-trideutero-3-(3,4-dihydroxyphenyl)-propionsäure-perdeuteroethylester,
L-2-Amino-2,3,3-trideutero-3-(3,4-dihydroxyphenyl)-propionsäure-perdeuterocyclohexylester,
L-2-Amino-2,3,3-trideutero-3-(2,3,6-trideutero-4,5-dihydroxyphenyl)propionsäure,
L-2-Amino-2,3,3-trideutero-3-(2,3,6-trideutero-4,5-dihydroxyphenyl)propionsäuremethylester,
L-2-Amino-2,3,3-trideutero-3-(2,3,6-trideutero-4,5-dihydroxyphenyl)propionsäureethylester,
L-2-Amino-2,3,3-trideutero-3-(2,3,6-trideutero-4,5-dihydroxyphenyl)propionsäurecyclohexylester,
L-2-Amino-2,3,3-trideutero-3-(2,3,6-trideutero-4,5-dihydroxyphenyl)propionsäure-perdeuteromethylester,
L-2-Amino-2,3,3-trideutero-3-(2,3,6-trideutero-4,5-dihydroxyphenyl)propionsäure-perdeuteroethylester,
L-2-Amino-2,3,3-trideutero-3-(2,3,6-trideutero-4,5-dihydroxyphenyl)propionsäure-perdeuterocyclohexylester,
L-2-Amino-2,3,3-trideutero-3-(2,3,6-trideutero-4,5-dideuteroxyphenyl)propionsäure-perdeuterocyclohexylester oder
L-2-Amino-3,3-dideutero-3-(4,5-dideuteroxyphenyl)-propionsäure-perdeuterocyclohexylester.

12. Verwendung der deuterierten Catecholaminderivate gemäß einem der Ansprüche 1 bis 11 sowie deren physiologisch verträglicher Salze, zur Herstellung von Arzneimitteln zur Behandlung von Dopaminmangelerkrankungen bzw. Erkrankungen die auf gestörtem Tyrosintransport oder gestörter Tyrosindecarboxylase beruhen wie Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie.

13. Verwendung der deuterierten Catecholaminderivate gemäß einem der Ansprüche 1 bis 11 sowie deren physiologisch verträglicher Salze, in Kombination mit einem Enzymhemmer oder mehreren Enzymhemmern, zur Herstellung von Arzneimitteln zur Behandlung von Dopaminmangelerkrankungen bzw. Erkrankungen die auf gestörtem Tyrosintransport oder gestörter Tyrosindecarboxylase beruhen wie Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie.

14. Verwendung der deuterierten Catecholaminderivate gemäß Anspruch 13 sowie deren physiologisch verträglicher Salze, **dadurch gekennzeichnet, dass** es sich bei dem Enzymhemmer bzw. den Enzymhemmern um Decarboxylasehemmer und/oder Catechol-O-Methyltransferase-Hemmer und/oder Monoaminoxidase-Hemmer und/oder β-Hydroxylase-Hemmer handelt.

15. Verwendung der deuterierten Catecholaminderivate gemäß Anspruch 14 sowie deren physiologisch verträglicher Salze, **dadurch gekennzeichnet, dass** der Decarboxylasehemmer ausgewählt wird aus der Gruppe, bestehend aus D,L-Serin-2-(2,3,4-trihydroxybenzyl)-hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

16. Verwendung der deuterierten Catecholaminderivate gemäß Anspruch 14 sowie deren physiologisch verträglicher Salze, **dadurch gekennzeichnet, dass** der Catechol-O-Methyltransferase Hemmer ausgewählt wird aus Entacapon und Cabergolin sowie deren physiologisch verträglicher Salze.

17. Verwendung der deuterierten Catecholaminderivate gemäß Anspruch 14 sowie deren physiologisch verträglicher Salze, **dadurch gekennzeichnet, dass** der Monoaminoxidase-Hemmer ausgewählt wird aus der Gruppe, bestehend aus Selegilin, Moclobemid und Tranylcypromin sowie deren physiologisch verträglicher Salze.

18. Verwendung der deuterierten Catecholaminderivate gemäß Anspruch 14 sowie deren physiologisch verträglicher Salze, **dadurch gekennzeichnet, dass** der β-Hydroxylase-Hemmer ausgewählt wird aus Calcium-5-butylpicolinat und Calcium-5-pentylpicolinat sowie deren physiologisch verträglicher Salze.

19. Verwendung der deuterierten Catecholaminderivate gemäß einem der Ansprüche 1-11 sowie deren physiologisch verträglicher Salze zur Herstellung von Arzneimitteln zur Behandlung von Morbus Parkinson, des Restless-Legs-Syndroms, von amyotrophischer Lateralsklerose und von multipler Systematrophie.

20. Pharmazeutische Zusammensetzung, welche deuterierte Catecholamine gemäß einem der Ansprüche 1-11 sowie deren physiologisch verträgliche Salze zur Behandlung von Morbus Parkinson, des Restless-Legs-Syndroms, von Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie, neben pharmazeutisch verträglichen Hilfs- und Zusatzstoffen, enthält.

21. Pharmazeutische Zusammensetzung, welche deuterierte Catecholamine gemäß einem der Ansprüche 1-11 sowie deren physiologisch verträgliche Salze zur Behandlung von Morbus Parkinson, Restless-Legs-Syndrom, Dystonie, zur Hemmung der Prolactinsekretion, zur Stimulierung der Wachstumshormon-Ausschüttung, zur Behandlung der neurologischen Symptome chronischer Manganvergiftungen, von amyotrophischer Lateralsklerose und von multipler Systematrophie, sowie einen oder mehrere Enzymhemmer, neben pharmazeutisch verträglichen Hilfs- und Zusatzstoffen, enthält.

22. Pharmazeutische Zusammensetzung gemäß Anspruch 21, **dadurch gekennzeichnet, dass** es sich bei dem Enzymhemmer bzw. den Enzymhemmern um Decarboxylasehemmer und/oder Catechol-O-Methyltransferase-Hemmer und/oder Monoaminoxidase-Hemmer und/oder β-Hydroxylase-Hemmer handelt.

23. Pharmazeutische Zusammensetzung gemäß Anspruch 21, **dadurch gekennzeichnet, dass** der Decarboxylasehemmer ausgewählt wird aus der Gruppe, bestehend aus D,L-Serin-2-(2,3,4-trihydroxybenzyl)hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

24. Pharmazeutische Zusammensetzung gemäß Anspruch 21, **dadurch gekennzeichnet, dass** der Catechol-O-Methyltransferase-Hemmer ausgewählt wird aus Entacapon und Cabergolin sowie deren physiologisch verträglicher Salze.

25. Pharmazeutische Zusammensetzung gemäß Anspruch 21, **dadurch gekennzeichnet, dass** der Monoaminoxidase-Hemmer ausgewählt wird aus der Gruppe, bestehend aus Selegilin, Moclobemid und Tranylcypromin sowie deren physiologisch verträglicher Salze.

26. Pharmazeutische Zusammensetzung gemäß Anspruch 21, **dadurch gekennzeichnet, dass** der β-Hydroxylase-Hemmer ausgewählt wird aus Calcium-5-butylpicolinat und Calcium-5-pentylpicolinat sowie deren physiologisch verträglicher Salze.

27. Verwendung der deuterierten Catecholaminderivate gemäß einem der Ansprüche 1 bis 11 sowie deren physiologisch verträglicher Salze, zur Herstellung von Arzneimitteln zur Prophylaxe von Psychosen insbesondere auch der Schizophrenie sowie zur Behandlung von akuten Psychosen, insbesondere bei Negativsymptomatik und insbesondere auch Schizophrenie.

28. Verwendung der deuterierten Catecholaminderivate gemäß einem der Ansprüche 1 bis 11 sowie deren physiologisch verträglicher Salze in Kombination mit einem oder mehreren Enzymhemmer, zur Herstellung von Arzneimitteln zur Prophylaxe von Psychosen sowie zur Behandlung von akuten Psychosen, insbesondere bei Negativsymptomatik.

29. Verwendung der deuterierten Catecholaminderivate gemäß Anspruch 28 sowie deren physiologisch verträglicher Salze, **dadurch gekennzeichnet, dass** es sich bei dem Enzymhemmer bzw. den Enzymhemmern um Decarboxylasehemmer und/oder Catechol-O-Methyltransferase-Hemmer und/oder Monoaminoxidase-Hemmer und/oder β-Hydroxylase-Hemmer handelt.

30. Verwendung der deuterierten Catecholaminderivate gemäß Anspruch 29 sowie deren physiologisch verträglicher Salze, **dadurch gekennzeichnet, dass** der Decarboxylasehemmer ausgewählt wird aus der Gruppe, bestehend aus D,L-Serin-2-(2,3,4-trihydroxybenzyl)-hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

31. Verwendung der deuterierten Catecholaminderivate gemäß Anspruch 29 sowie deren physiologisch verträglicher Salze, **dadurch gekennzeichnet, dass** der Catechol-O-Methyltransferase-Hemmer ausgewählt wird aus Entacapon und Cabergolin sowie deren physiologisch verträglicher Salze.

32. Verwendung der deuterierten Catecholaminderivate gemäß Anspruch 29 sowie deren physiologisch verträglicher Salze, **dadurch gekennzeichnet, dass** der Monoaminoxidase-Hemmer ausgewählt wird aus der Gruppe, bestehend aus Selegilin, Moclobemid und Tranylcypromin sowie deren physiologisch verträglicher Salze.

33. Verwendung der deuterierten Catecholaminderivate gemäß Anspruch 29 sowie deren physiologisch verträglicher Salze, **dadurch gekennzeichnet, dass** der β-Hydroxylase-Hemmer ausgewählt wird aus Calcium-5-butylpicolinat und Calcium-5-pentylpicolinat sowie deren physiologisch verträglicher Salze.

34. Verwendung der deuterierten Catecholaminderivate gemäß einem der Ansprüche 1-11 sowie deren physiologisch verträglicher Salze zur Herstellung von Arzneimitteln zur Prophylaxe von Psychosen sowie zur Behandlung von akuten Psychosen, insbesondere bei Negativsymptomatik.

35. Pharmazeutische Zusammensetzung, welche deuterierte Catecholamine gemäß einem der Ansprüche 1-11 sowie deren physiologisch verträgliche Salze zur Prophylaxe von Psychosen sowie zur Behandlung von akuten Psychosen, insbesondere bei Negativsymptomatik, neben pharmazeutisch verträglichen Hilfs- und Zusatzstoffen, enthält.

36. Pharmazeutische Zusammensetzung, welche deuterierte Catecholamine gemäß einem der Ansprüche 1-11 sowie deren physiologisch verträgliche Salze zur Prophylaxe von Psychosen und zur Behandlung von akuten Psychosen, insbesondere bei Negativsymptomatik sowie einen oder mehrere Enzymhemmer, neben pharmazeutisch verträglichen Hilfs- und Zusatzstoffen, enthält.

37. Pharmazeutische Zusammensetzung gemäß Anspruch 36, **dadurch gekennzeichnet, dass** es sich bei dem Enzymhemmer bzw. den Enzymhemmern um Decarboxylasehemmer und/oder Catechol-O-Methyltransferase-Hemmer und/oder Monoaminoxidase-Hemmer und/oder β-Hydroxylase-Hemmer handelt.

38. Pharmazeutische Zusammensetzung gemäß Anspruch 37, **dadurch gekennzeichnet, dass** der Decarboxylasehemmer ausgewählt wird aus der Gruppe, bestehend aus D,L-Serin-2-(2,3,4-trihydroxybenzyl)hydrazid (Benserazid), (-)-L-α-Hydrazino-3,4-dihydroxy-α-methylhydrozimtsäure (Carbidopa), L-Serin-2-(2,3,4-trihydroxybenzyl)hydrazid, Glycin-2-(2,3,4-trihydroxybenzyl)hydrazid und L-Tyrosin-2-(2,3,4-trihydroxybenzyl)hydrazid sowie deren physiologisch verträglicher Salze.

39. Pharmazeutische Zusammensetzung gemäß Anspruch 37, **dadurch gekennzeichnet, dass** der Catechol-O-Methyltransferase-Hemmer ausgewählt wird aus Entacapon und Cabergolin sowie deren physiologisch verträglicher Salze.

40. Pharmazeutische Zusammensetzung gemäß Anspruch 37, **dadurch gekennzeichnet, dass** der Monoaminoxidase-Hemmer ausgewählt wird aus der Gruppe, bestehend aus Selegilin, Moclobemid und Tranylcypromin sowie deren physiologisch verträglicher Salze.

41. Pharmazeutische Zusammensetzung gemäß Anspruch 37, **dadurch gekennzeichnet, dass** der β-Hydroxylase-Hemmer ausgewählt wird aus Calcium-5-butylpicolinat und Calcium-5-pentylpicolinat sowie deren physiologisch verträglicher Salze.

## Claims

1. Deuterated catecholamine derivatives of the general formula I 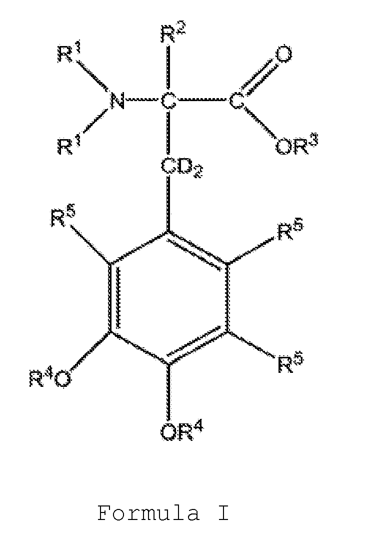 wherein
R¹ is H or D, R² indicates H or D, R³ is H, D,
C₁-C₆-alkyl or C₅ to C₆-cycloalkyl, deuterated C₁ to C₆-alkyl or C₅ to C**₆**-cycloalkyl, R⁴ indicates H or D and R⁵ is H or D, with the exception of L-2-amino-3,3-dideutero-3-(3,4-dihydroxyphenyl) propionic acid.

2. Deuterated catecholamine derivatives according to claim 1, wherein R¹ is H or D, R² indicates H or D, R³ is H, D, C₁ to C₆-alkyl or C₅ to C₆-cycloalkyl, deuterated C₁ to C₆-alkyl or deuterated C₅ to C₆-cycloalkyl, R⁴ indicates H or D and R⁵ is D.

3. Deuterated catecholamine derivatives according to claim 1, wherein R¹ is H or D, R² indicates D, R³ is D, C₁ to C₆-alkyl or C₅ to C₆-cycloalkyl, deuterated C₁ to C₆-alkyl or deuterated C₅ to C₆-cycloalkyl, R⁴ indicates H or D and R⁵ is D.

4. Deuterated catecholamine derivatives according to claim 1, wherein R¹ is H or D, R² indicates D, R³ is H, D, C₁ to C₆-alkyl or C₅ to C₆-cycloalkyl, deuterated C₁ to C₆-alkyl or deuterated C₅ to C₆-cycloalkyl, R⁴ indicates H or D and R⁵ is D.

5. Deuterated catecholamine derivatives according to general formula I, wherein R¹ is H or D, R² indicates D, R³ is C₁ to C₆-alkyl or C₅ to C₆-cycloalkyl, R⁴ indicates H or D and R⁵ is D.

6. Deuterated catecholamine derivatives according to claim 1, wherein R¹ is H or D, R² indicates D, R³ is methyl, R⁴ indicates H or D and R⁵ is D.

7. Deuterated catecholamine derivatives according to claim 1, wherein R¹ is H or D, R² indicates D, R³ is ethyl, R⁴ indicates H or D and R⁵ is D.

8. Deuterated catecholamine derivatives according to claim 1, wherein R¹ is H or D, R² indicates D, R³ is perdeuteroethyl, R⁴ indicates H or D and R⁵ is D.

9. Deuterated catecholamine derivatives according to claim 1, wherein R¹ is H or D, R² indicates H or D, R³ is perdeuteroethyl, R⁴ indicates H or D and R⁵ is D.

10. Deuterated catecholamine derivatives according to claim 1, wherein R¹ is H or D, R² indicates H or D, R³ is perdeuteroethyl, R⁴ indicates D and R⁵ is H or D.

11. Deuterated catecholamine derivatives according to claim 1, namely
L-2-amino-3,3-dideutero-3-(3,4-dihydroxyphenyl) methyl propionate,
L-2-amino-3,3-dideutero-3-(3,4-dihydroxyphenyl) ethyl propionate,
L-2-amino-3,3-dideutero-3-(3,4-dihydroxyphenyl) cyclohexyl propionate,
L-2-amino-3,3-dideutero-3-(3,4-dihydroxyphenyl) perdeuteromethyl propionate,
L-2-amino-3,3-dideutero-3-(3,4-dihydroxyphenyl) perdeuteroethyl propionate,
L-2-amino-3,3-dideutero-3-(3,4-dihydroxyphenyl) perdeuterocyclohexyl propionate,
L-2-amino-2,3,3-trideutero-3-(3,4-dihydroxyphenyl) propionic acid,
L-2-amino-2,3,3-trideutero-3-(3,4-dihydroxyphenyl) methyl propionate,
L-2-amino-2,3,3-trideutero-3-(3,4-dihydroxyphenyl) ethyl propionate,
L-2-amino-2,3,3-trideutero-3-(3,4-dihydroxyphenyl) cyclohexyl propionate,
L-2-amino-2,3,3-trideutero-3-(3,4-dihydroxyphenyl) perdeuteromethyl propionate,
L-2-amino-2,3,3-trideutero-3-(3,4-dihydroxyphenyl) perdeuteroethyl propionate,
L-2-amino-2,3,3-trideutero-3-(3,4-dihydroxyphenyl) perdeuterocyclohexyl propionate,
L-2-amino-2,3,3-trideutero-3-(2,3,6-trideutero-4,5-dihydroxyphenyl) propionic acid,
L-2-amino-2,3,3-trideutero-3-(2,3,6-trideutero-4,5-dihydroxyphenyl) methyl propionate,
L-2-amino-2,3,3-trideutero-3-(2,3,6-trideutero-4,5-dihydroxyphenyl) ethyl propionate,
L-2-amino-2,3,3-trideutero-3-(2,3,6-trideutero-4,5-dihydroxyphenyl) cyclohexyl propionate,
L-2-amino-2,3,3-trideutero-3-(2,3,6-trideutero-4,5-dihydroxyphenyl) perdeuteromethyl propionate,
L-2-amino-2,3,3-trideutero-3-(2,3,6-trideutero-4,5-dihydroxyphenyl) perdeuteroethyl propionate,
L-2-amino-2,3,3-trideutero-3-(2,3,6-trideutero-4,5-dihydroxyphenyl) perdeuterocyclohexyl propionate,
L-2-amino-2,3,3-trideutero-3-(2,3,6-trideutero-4,5-dideuteroxyphenyl) perdeuterocyclohexyl propionate,
L-2-amino-3,3-dideutero-3-(4,5-dideuteroxyphenyl) perdeuterocyclohexyl propionate.

12. Use of the deuterated catecholamine derivatives according to one of claims 1 to 11 as well as the physiologically compatible salts thereof,
for the production of pharmaceuticals for the treatment of dopamine deficiency diseases or diseases which are based on disrupted tyrosine transport or disrupted tyrosine decarboxylase, such as Parkinson's disease, restless leg syndrome, dystonia, for inhibiting prolactin secretion, for stimulating the release of growth hormone, for the treatment of neurological symptoms of chronic manganese intoxications, of amyotrophic lateral sclerosis and of multiple system atrophy.

13. Use of the deuterated catecholamine derivatives according to one of claims 1 to 11 as well as the physiologically compatible salts thereof, in combination with an enzyme inhibitor or several enzyme inhibitors, for the production of pharmaceuticals for the treatment of dopamine deficiency diseases or diseases which are based on disrupted tyrosine transport or disrupted tyrosine decarboxylase, such as Parkinson's disease, restless leg syndrome, dystonia, for inhibiting prolactin secretion, for stimulating the release of growth hormone, for the treatment of neurological symptoms of chronic manganese intoxications, of amyotrophic lateral sclerosis and of multiple system atrophy.

14. Use of deuterated catecholamine derivatives according to claim 13 as well as the physiologically compatible salts thereof, **characterized in that** the enzyme inhibitor or the enzyme inhibitors are decarboxylase inhibitors and/or catechol-0-methyltransferase inhibitors and/or monoamine oxidase inhibitors and/or β-hydroxylase inhibitors.

15. Use of deuterated catecholamine derivatives according to claim 14 as well as the physiologically compatible salts thereof, **characterized in that** the decarboxylase inhibitor is selected from the group consisting of D,L-serine 2-(2,3,4-trihydroxybenzyl) hydrazide (benserazide), (-)-L-α-hydrazino-3,4-dihydroxy-α-methylhydrocinnamic acid (carbidopa), L-serine 2-(2,3,4-trihydroxybenzyl) hydrazide, glycine 2-(2,3,4-trihydroxybenzyl) hydrazide and L-tyrosine 2-(2,3,4-trihydroxybenzyl) hydrazide as well as the physiologically compatible salts thereof.

16. Use of the deuterated catecholamine derivatives according to claim 14 as well as the physiologically compatible salts thereof, **characterized in that** the catechol-0-methyltransferase inhibitor is selected from entacapone and cabergoline as well as the physiologically compatible salts thereof.

17. Use of the deuterated catecholamine derivatives according to claim 14 as well as the physiologically compatible salts thereof, **characterized in that** the monoamine oxidase inhibitor is selected from the group consisting of selegiline, moclobemide and tranylcypromine as well as the physiologically compatible salts thereof.

18. Use of the deuterated catecholamine derivatives according to claim 14 as well as the physiologically compatible salts thereof, **characterized in that** the β-hydroxylase inhibitor is selected from calcium 5-butyl picolinate and calcium 5-pentyl picolinate as well as the physiologically compatible salts thereof.

19. Use of the deuterated catecholamine derivatives according to one of claims 1 to 11 as well as the physiologically compatible salts thereof, for the production of pharmaceuticals for the treatment of Parkinson's disease, restless leg syndrome, of amyotrophic lateral sclerosis and of multiple system atrophy.

20. A pharmaceutical composition, which contains deuterated catecholamines according to one of claims 1 to 11 as well as the physiologically compatible salts thereof, for the treatment of Parkinson's disease, of restless leg syndrome, of dystonia, for inhibiting prolactin secretion, for stimulating the release of growth hormone, for the treatment of neurological symptoms of chronic manganese intoxications, of amyotrophic lateral sclerosis and of multiple system atrophy, in addition to pharmaceutically compatible adjuvants and additives.

21. A pharmaceutical composition, which contains deuterated catecholamines according to one of claims 1 to 11 as well as the physiologically compatible salts thereof, for the treatment of Parkinson's disease, restless leg syndrome, dystonia, for inhibiting prolactin secretion, for stimulating the release of growth hormone, for the treatment of neurological symptoms of chronic manganese intoxications, of amyotrophic lateral sclerosis and of multiple system atrophy, as well as one or more enzyme inhibitors, in addition to pharmaceutically compatible adjuvants and additives.

22. The pharmaceutical composition according to claim 21, **characterized in that** the enzyme inhibitor is or the enzyme inhibitors are decarboxylase inhibitors and/or catechol-0-methyltransferase inhibitors and/or monoamine oxidase inhibitors and/or β-hydroxylase inhibitors.

23. The pharmaceutical composition according to claim 21, **characterized in that** the decarboxylase inhibitor is selected from the group consisting of D,L-serine 2-(2,3,4-trihydroxybenzyl) hydrazide (benserazide), (-)-L-α-hydrazino-3,4-dihydroxy-α-methylhydrocinnamic acid (carbidopa), L-serine 2-(2,3,4-trihydroxybenzyl) hydrazide, glycine 2-(2,3,4-trihydroxybenzyl) hydrazide and L-tyrosine 2-(2,3,4-trihydroxybenzyl) hydrazide as well as the the physiologically compatible salts thereof.

24. The pharmaceutical composition according to claim 21, **characterized in that** the catechol-0-methyltransferase inhibitor is selected from entacapone and cabergoline as well as the physiologically compatible salts thereof.

25. The pharmaceutical composition according to claim 21, **characterized in that** the monoamine oxidase inhibitor is selected from the group consisting of selegiline, moclobemide and tranylcypromine as well as the physiologically compatible salts thereof.

26. The pharmaceutical composition according to claim 21, **characterized in that** the β-hydroxylase inhibitor is selected from calcium 5-butyl picolinate and calcium 5-pentyl picolinate as well as the physiologically compatible salts thereof.

27. Use of the deuterated catecholamine derivatives according to one of claims 1 to 11 as well as the physiologically compatible salts thereof, for the production of pharmaceuticals for the prophylaxis of psychoses, particularly also of schizophrenia, as well as for the treatment of acute psychoses, particularly in the case of negative symptomatology and particularly also schizophrenia.

28. Use of the deuterated catecholamine derivatives according to one of claims 1 to 11 as well as the physiologically compatible salts thereof, in combination with one or more enzyme inhibitors, for the production of pharmaceuticals for the prophylaxis of psychoses, as well as for the treatment of acute psychoses, particularly in the case of negative symptomatology.

29. Use of the deuterated catecholamine derivatives according to claim 28 as well as the physiologically compatible salts thereof, **characterized in that** the enzyme inhibitor or the enzyme inhibitors involve decarboxylase inhibitors and/or catechol-0-methyltransferase inhibitors and/or monoamine oxidase inhibitors and/or β-hydroxylase inhibitors.

30. Use of the deuterated catecholamine derivatives according to claim 29 as well as the physiologically compatible salts thereof, **characterized in that** the decarboxylase inhibitor is selected from the group consisting of D,L-serine 2-(2,3,4-trihydroxybenzyl) hydrazide (benserazide), (-)-L-α-hydrazino-3,4-dihydroxy-α-methylhydrocinnamic acid (carbidopa), L-serine 2-(2,3,4-trihydroxybenzyl) hydrazide, glycine 2-(2,3,4-trihydroxybenzyl) hydrazide and L-tyrosine 2-(2,3,4-trihydroxybenzyl) hydrazide as well as the physiologically compatible salts thereof.

31. Use of the deuterated catecholamine derivatives according to claim 29 as well as the physiologically compatible salts thereof, **characterized in that** the catechol-0-methyltransferase inhibitor is selected from entacapone and cabergoline as well as the physiologically compatible salts thereof.

32. Use of the deuterated catecholamine derivatives according to claim 29 as well as the physiologically compatible salts thereof, **characterized in that** the monoamine oxidase inhibitor is selected from the group, consisting of selegiline, moclobemide and tranylcypromine as well as the physiologically compatible salts thereof.

33. Use of the deuterated catecholamine derivatives according to claim 29 as well as the physiologically compatible salts thereof, **characterized in that** the β-hydroxylase inhibitor is selected from calcium 5-butyl picolinate and calcium 5-pentyl picolinate as well as the physiologically compatible salts thereof.

34. Use of the deuterated catecholamine derivatives according to one of claims 1-11 as well as the physiologically compatible salts thereof, for the production of pharmaceuticals for the prophylaxis of psychoses as well as for the treatment of acute psychoses, particularly in the case of negative symptomatology.

35. A pharmaceutical composition, which contains deuterated catecholamines according to one of claims 1-11 as well as the physiologically compatible salts thereof, for the prophylaxis of psychoses as well as for the treatment of acute psychoses, particularly in the case of negative symptomatology, in addition to pharmaceutically compatible adjuvants and additives.

36. Pharmaceutical composition, which contains deuterated catecholamine derivatives according to one of claims 1-11 as well as the physiologically compatible salts thereof, for the prophylaxis of psychoses and for the treatment of acute psychoses, particularly in the case of negative symptomatology, as well as one or more enzyme inhibitors, in addition to pharmaceutically compatible adjuvants and additives.

37. The pharmaceutical composition according to claim 36, **characterized in that** the enzyme inhibitor or the enzyme inhibitors involve decarboxylase inhibitors and/or catechol-0-methyltransferase inhibitors and/or monoamine oxidase inhibitors and/or β-hydroxylase inhibitors.

38. The pharmaceutical composition according to claim 37, **characterized in that** the decarboxylase inhibitor is selected from the group consisting of D,L-serine 2-(2,3,4-trihydroxybenzyl) hydrazide (benserazide), (-)-L-α-hydrazino-3,4-dihydroxy-α-methylhydrocinnamic acid (carbidopa), L-serine 2-(2,3,4-trihydroxybenzyl) hydrazide, glycine 2-(2,3,4-trihydroxybenzyl) hydrazide and L-tyrosine 2-(2,3,4-trihydroxybenzyl) hydrazide as well as the physiologically compatible salts thereof.

39. The pharmaceutical composition according to claim 37, **characterized in that** the catechol-0-methyltransferase inhibitor is selected from entacapone and cabergoline as well as the physiologically compatible salts thereof.

40. The pharmaceutical composition according to claim 37, **characterized in that** the monoamine oxidase inhibitor is selected from the group consisting of selegiline, moclobemide and tranylcypromine as well as the physiologically compatible salts thereof.

41. The pharmaceutical composition according to claim 37, **characterized in that** the β-hydroxylase inhibitor is selected from calcium 5-butyl picolinate and calcium 5-pentyl picolinate as well as the physiologically compatible salts thereof.

## Revendications

1. Dérivés deutérés de catécholamine de formule générale I, 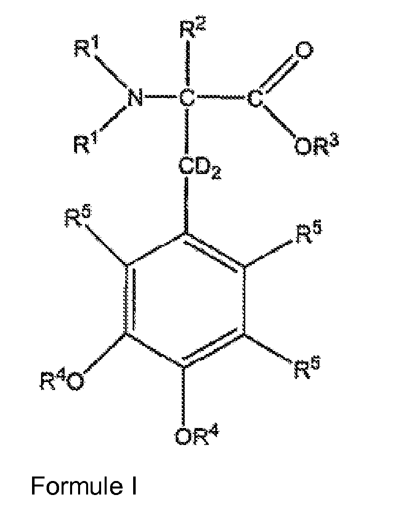 où
R¹ représente H ou D, R² signifie H ou D, R³ représente H, D, C₁-C₆-alkyle ou C₅-C₆-cycloalkyle, C₁-C₆-alkyle deutéré ou C₅-C₆-cydoalkyle deutéré, R⁴ signifie H ou D et R⁵ représente H ou D, à l'exception de l'acide L-2-amino-3,3-dideutéro-3-(3,4-dihydroxyphényl)-propionique.

2. Dérivés deutérés de catécholamine selon la revendication 1, où R¹ représente H ou D, R² signifie H ou D, R³ représente H, D, C₁-C₆-alkyle ou C₅-C₆-cycloalkyle, C₁-C₆-alkyle deutéré ou C₅-C₆-cydoalkyle deutéré, R⁴ signifie H ou D et R⁵ représente D.

3. Dérivés deutérés de catécholamine selon la revendication 1, où R¹ représente H ou D, R² signifie D, R³ représente D, C₁-C₆-alkyle ou C₅-C₆-cycloalkyle, C₁-C₆-alkyle deutéré ou C₅-C₆-cydoalkyle deutéré, R⁴ signifie H ou D et R⁵ représente D.

4. Dérivés deutérés de catécholamine selon la revendication 1, où R¹ représente H ou D, R² signifie D, R³ représente H, D, C₁-C₆-alkyle ou C₅-C₆-cycloalkyle, C₁-C₆-alkyle deutéré ou C₅-C₆-cydoalkyle deutéré, R⁴ signifie H ou D et R⁵ représente D.

5. Dérivés deutérés de catécholamine selon la formule générale I, où R¹ représente H ou D, R² signifie D, R³ représente C₁-C₆-alkyle ou C₅-C₆-cycloalkyle, R⁴ signifie H ou D et R⁵ représente D.

6. Dérivés deutérés de catécholamine selon la revendication 1, où R¹ représente H ou D, R² signifie D, R³ représente méthyle, R⁴ signifie H ou D et R⁵ représente D.

7. Dérivés deutérés de catécholamine selon la revendication 1, où R¹ représente H ou D, R² signifie D, R³ représente éthyle, R⁴ signifie H ou D et R⁵ représente D.

8. Dérivés deutérés de catécholamine selon la revendication 1, où R¹ représente H ou D, R² signifie D, R³ représente perdeutéroéthyle, R⁴ signifie H ou D et R⁵ représente D.

9. Dérivés deutérés de catécholamine selon la revendication 1, où R¹ représente H ou D, R² signifie H ou D, R³ représente perdeutéroéthyle, R⁴ signifie H ou D et R⁵ représente D.

10. Dérivés deutérés de catécholamine selon la revendication 1, où R¹ représente H ou D, R² signifie H ou D, R³ représente perdeutéroéthyle, R⁴ signifie D et R⁵ représente H ou D.

11. Dérivés deutérés de catécholamine selon la revendication 1, notamment
l'ester méthylique de l'acide L-2-amino-3,3-dideutéro-3-(3,4-dihydroxyphényl)-propionique,
l'ester éthylique de l'acide L-2-amino-3,3-dideutéro-3-(3,4-dihydroxyphényl)-propionique,
**l'ester cyclohexylique de l'acide** L-2-amino-3,3-dideutéro-3-(3,4-dihydroxyphényl)-propionique,
l'ester perdeutérométhylique de l'acide L-2-amino-3,3-dideutéro-3-(3,4-dihydroxyphényl)-propionique,
l'ester perdeutéroéthylique de l'acide L-2-amino-3,3-dideutéro-3-(3,4-dihydroxyphényl)-propionique,
l'ester perdeutérocyclohexylique de l'acide L-2-amino-3,3-dideutéro-3-(3,4-dihydroxyphényl)-propionique,
l'acide L-2-amino-2,3,3-trideutéro-3-(3,4-dihydroxyphényl)-propionique,
l'ester méthylique de l'acide L-2-amino-2,3,3-trideutéro-3-(3,4-dihydroxyphényl)-propionique,
l'ester éthylique de l'acide L-2-amino-2,3,3-trideutéro-3-(3,4-dihydroxyphényl)-propionique,
**l'ester cyclohexylique de l'acide** L-2-amino-2,3,3-trideutéro-3-(3,4-dihydroxyphényl)-propionique,
l'ester perdeutérométhylique de l'acide L-2-amino-2,3,3-trideutéro-3-(3,4-dihydroxyphényl)-propionique,
l'ester perdeutéroéthylique de l'acide L-2-amino-2,3,3-trideutéro-3-(3,4-dihydroxyphényl)-propionique,
l'ester perdeutérocyclohexylique de l'acide L-2-amino-2,3,3-trideutéro-3-(3,4-dihydroxyphényl)-propionique,
**l'acide** L-2-amino-2,3,3-trideutéro-3-(2,3,6-trideutéro-4,5-dihydroxyphényl)propionique,
l'ester méthylique de l'acide L-2-amino-2,3,3-trideutéro-3-(2,3,6-trideutéro-4,5-dihydroxyphényl)propionique,
l'ester éthylique de l'acide L-2-amino-2,3,3-trideutéro-3-(2,3,6-trideutéro-4,5-dihydroxyphényl)propionique,
l'ester cyclohexylique de l'acide L-2-amino-2,3,3-trideutéro-3-(2,3,6-trideutéro-4,5-dihydroxyphényl)propionique,
l'ester perdeutérométhylique de l'acide L-2-amino-2,3,3-trideutéro-3-(2,3,6-trideutéro-4,5-dihydroxyphényl)propionique,
l'ester perdeutéroéthylique de l'acide L-2-amino-2,3,3-trideutéro-3-(2,3,6-trideutéro-4,5-dihydroxyphényl)propionique,
l'ester perdeutérocyclohexylique de l'acide L-2-amino-2,3,3-trideutéro-3-(2,3,6-trideutéro-4,5-dihydroxyphényl)propionique,
l'ester perdeutérocyclohexylique de l'acide L-2-amino-2,3,3-trideutéro-3-(2,3,6-trideutéro-4,5-dideutéroxyphényl)propionique ou
l'ester perdeutérocyclohexylique de l'acide L-2-amino-3,3-dideutéro-3-(4,5-dideutéroxyphényl)-propionique.

12. Utilisation des dérivés deutérés de la catécholamine selon l'une quelconque des revendications 1 à 11 ainsi que de leurs sels physiologiquement acceptables, pour la préparation de médicaments destinés au traitement des maladies liées aux carences en dopamine, ou, selon le cas, de maladies qui reposent sur un transport perturbé de la tyrosine ou une tyrosine décarboxylase perturbée, telles que la maladie de Parkinson, le syndrome des jambes sans repos, la dystonie, à l'inhibition de la sécrétion de prolactine, à la stimulation de l'excrétion de l'hormone de croissance, au traitement des symptômes neurologiques d'empoisonnements chroniques au manganèse, de la sclérose latérale amyotrophique et de l'atrophie multisystématisée.

13. Utilisation des dérivés deutérés de la catécholamine selon l'une quelconque des revendications 1 à 11 ainsi que de leurs sels physiologiquement acceptables, en combinaison avec un inhibiteur d'enzymes ou plusieurs inhibiteurs d'enzymes pour la préparation de médicaments destinés au traitement des maladies liées aux carences en dopamine, ou, selon le cas, de maladies qui reposent sur un transport perturbé de la tyrosine ou une tyrosine décarboxylase perturbée, telles que la maladie de Parkinson, le syndrome des jambes sans repos, la dystonie, à l'inhibition de la sécrétion de prolactine, à la stimulation de l'excrétion de l'hormone de croissance, au traitement des symptômes neurologiques d'empoisonnements chroniques au manganèse, de la sclérose latérale amyotrophique et de l'atrophie multisystématisée.

14. Utilisation des dérivés deutérés de la catécholamine selon la revendication 13 ainsi que de leurs sels physiologiquement acceptables **caractérisée en ce qu'**il s'agit, pour l'inhibiteur d'enzymes ou les inhibiteurs d'enzymes d'un inhibiteur de la décarboxylase et/ou d'un inhibiteur de la catéchol-O-méthyltransférase et/ou d'un inhibiteur de la monoaminoxydase et/ou d'un inhibiteur de la β-hydroxylase.

15. Utilisation des dérivés deutérés de la catécholamine selon la revendication 14 ainsi que de leurs sels physiologiquement acceptables **caractérisée en ce que** l'inhibiteur de la décarboxylase est choisi dans le groupe constitué par le D,L-sérine-2-(2,3,4-trihydroxybenzyl)-hydrazide (Benserazid), l'acide (-)-L-α-hydrazino-3,4-dihydroxy-α-méthylhydrocinnamique (Carbidopa), **le** L-sérine-2-(2,3,4-trihydroxybenzyl)hydrazide, le glycine-2-(2,3,4-trihydroxybenzyl)hydrazide et le L-tyrosine-2-(2,3,4-trihydroxybenzyl)hydrazide ainsi que leurs sels physiologiquement acceptables.

16. Utilisation des dérivés deutérés de la catécholamine selon la revendication 14 ainsi que de leurs sels physiologiquement acceptables **caractérisée en ce que** l'inhibiteur de la catéchol-O-méthyltransférase est choisi parmi l'Entacapone et la Cabergoline ainsi que leurs sels physiologiquement acceptables.

17. Utilisation des dérivés deutérés de la catécholamine selon la revendication 14 ainsi que de leurs sels physiologiquement acceptables **caractérisée en ce que** l'inhibiteur de la monoaminoxydase est choisi dans le groupe constitué par la Sélégiline, la Moclobémide et la Tranylcypromine ainsi que leurs sels physiologiquement acceptables.

18. Utilisation des dérivés deutérés de la catécholamine selon la revendication 14 ainsi que de leurs sels physiologiquement acceptables **caractérisée en ce que** l'inhibiteur de la β-hydroxylase est choisi parmi le calcium-5-butylpicolinate et le calcium-5-pentylpicolinate ainsi que leurs sels physiologiquement acceptables.

19. Utilisation des dérivés deutérés de la catécholamine selon l'une quelconque des revendications 1 à 11 ainsi que de leurs sels physiologiquement acceptables, pour la préparation de médicaments destinés au traitement de la maladie de Parkinson, du syndrome des jambes sans repos, de la sclérose latérale amyotrophique et de l'atrophie multisystématisée.

20. Préparation pharmaceutique qui contient des catécholamines deutérées selon l'une quelconque des revendications 1 à 11 ainsi que leurs sels physiologiquement acceptables pour le traitement de la maladie de Parkinson, du syndrome des jambes sans repos, de la dystonie, pour l'inhibition de la sécrétion de la prolactine, pour la stimulation de l'excrétion de l'hormone de croissance, pour le traitement des syndromes neurologiques d'empoisonnements chroniques au manganèse, de la sclérose latérale amyotrophique et de l'atrophie multisystématisée, outre des additifs et adjuvants pharmaceutiquement acceptables.

21. Préparation pharmaceutique qui contient des catécholamines deutérées selon l'une quelconque des revendications 1 à 11 ainsi que leurs sels physiologiquement acceptables pour le traitement de la maladie de Parkinson, du syndrome des jambes sans repos, de la dystonie, pour l'inhibition de la sécrétion de la prolactine, pour la stimulation de l'excrétion de l'hormone de croissance, pour le traitement des syndromes neurologiques d'empoisonnements chroniques au manganèse, de la sclérose latérale amyotrophique et de l'atrophie multisystématisée ainsi qu'un ou plusieurs inhibiteurs des enzymes, outre des additifs et adjuvants pharmaceutiquement acceptables.

22. Préparation pharmaceutique selon la revendication 21, **caractérisée en ce qu'**il s'agit, pour l'inhibiteur d'enzymes ou les inhibiteurs d'enzymes d'un inhibiteur de la décarboxylase et/ou d'un inhibiteur de la catéchol-O-méthyltransférase et/ou d'un inhibiteur de la monoaminoxydase et/ou d'un inhibiteur de la β-hydroxylase.

23. Préparation pharmaceutique selon la revendication 21, **caractérisée en ce que** l'inhibiteur de la décarboxylase est choisi dans le groupe constitué par le D,L-sérine-2-(2,3,4-trihydroxybenzyl)-hydrazide (Benserazid), l'acide (-)-L-α-hydrazino-3,4-dihydroxy-α-méthylhydrocinnamique (Carbidopa), le L-sérine-2-(2,3,4-**trihydroxybenzyl)hydrazide, le glycine**-2-(2,3,4-trihydroxybenzyl)hydrazide et le L-tyrosine-2-(2,3,4-trihydroxybenzyl)hydrazide ainsi que leurs sels physiologiquement acceptables.

24. Préparation pharmaceutique selon la revendication 21, **caractérisée en ce que** l'inhibiteur de la catéchol-O-méthyltransférase est choisi parmi l'Entacapone et la Cabergoline ainsi que leurs sels physiologiquement acceptables.

25. Préparation pharmaceutique selon la revendication 21, **caractérisée en ce que** l'inhibiteur de la monoaminoxydase est choisi dans le groupe constitué par la Sélégiline, la Moclobémide et la Tranylcypromine ainsi que leurs sels physiologiquement acceptables.

26. Préparation pharmaceutique selon la revendication 21, **caractérisée en ce que** l'inhibiteur de la β-hydroxylase est choisi parmi le calcium-5-butylpicolinate et le calcium-5-pentylpicolinate ainsi que leurs sels physiologiquement acceptables.

27. Utilisation des dérivés deutérés de la catécholamine selon l'une quelconque des revendications 1 à 11 ainsi que de leurs sels physiologiquement acceptables, pour la préparation de médicaments destinés à la prophylaxie de psychoses, en particulier également la schizophrénie ainsi qu'au traitement de psychoses aiguës, en particulier lors de la symptomatique négative et en particulier également la schizophrénie.

28. Utilisation des dérivés deutérés de la catécholamine selon l'une quelconque des revendications 1 à 11 ainsi que de leurs sels physiologiquement acceptables, en combinaison avec un ou plusieurs inhibiteurs d'enzymes, pour la préparation de médicaments destinés à la prophylaxie de psychoses ainsi qu'au traitement de psychoses aiguës, en particulier lors de la symptomatique négative.

29. Utilisation des dérivés deutérés de la catécholamine selon la revendication 28 ainsi que de leurs sels physiologiquement acceptables **caractérisée en ce qu'**il s'agit, pour l'inhibiteur d'enzymes ou les inhibiteurs d'enzymes d'un inhibiteur de la décarboxylase et/ou d'un inhibiteur de la catéchol-O-méthyltransférase et/ou d'un inhibiteur de la monoaminoxydase et/ou d'un inhibiteur de la β-hydroxylase.

30. Utilisation des dérivés deutérés de la catécholamine selon la revendication 29 ainsi que de leurs sels physiologiquement acceptables **caractérisée en ce que** l'inhibiteur de la décarboxylase est choisi dans le groupe constitué par le D,L-sérine-2-(2,3,4-trihydroxybenzyl)-hydrazide (Benserazid), l'acide (-)-L-α-hydrazino-3,4-dihydroxy-α-méthylhydrocinnamique (Carbidopa), I e L-sérine-2-(2,3,4-trihydroxybenzyl)hydrazide, le glycine-2-(2,3,4-trihydroxybenzyl)hydrazide et le L-tyrosine-2-(2,3,4-trihydroxybenzyl)hydrazide ainsi que leurs sels physiologiquement acceptables.

31. Utilisation des dérivés deutérés de la catécholamine selon la revendication 29 ainsi que de leurs sels physiologiquement acceptables **caractérisée en ce que** l'inhibiteur de la catéchol-O-méthyltransférase est choisi parmi l'Entacapone et la Cabergoline ainsi que leurs sels physiologiquement acceptables.

32. Utilisation des dérivés deutérés de la catécholamine selon la revendication 29 ainsi que de leurs sels physiologiquement acceptables **caractérisée en ce que** l'inhibiteur de la monoaminoxydase est choisi dans le groupe constitué par la Sélégiline, la Moclobémide et la Tranylcypromine ainsi que leurs sels physiologiquement acceptables.

33. Utilisation des dérivés deutérés de la catécholamine selon la revendication 29 ainsi que de leurs sels physiologiquement acceptables **caractérisée en ce que** l'inhibiteur de la β-hydroxylase est choisi parmi le calcium-5-butylpicolinate et le calcium-5-pentylpicolinate ainsi que leurs sels physiologiquement acceptables.

34. Utilisation des dérivés deutérés de la catécholamine selon l'une quelconque des revendications 1 à 11 ainsi que de leurs sels physiologiquement acceptables, pour la préparation de médicaments destinés à la prophylaxie de psychoses ainsi qu'au traitement de psychoses aiguës, en particulier lors de la symptomatique négative.

35. Préparation pharmaceutique qui contient des catécholamines deutérées selon l'une quelconque des revendications 1 à 11 ainsi que leurs sels physiologiquement acceptables pour la prophylaxie de psychoses ainsi que pour le traitement de psychoses aiguës, en particulier lors de la symptomatique négative, outre des additifs et adjuvants pharmaceutiquement acceptables.

36. Préparation pharmaceutique qui contient des catécholamines deutérées selon l'une quelconque des revendications 1 à 11 ainsi que leurs sels physiologiquement acceptables pour la prophylaxie de psychoses et pour le traitement de psychoses aiguës, en particulier lors de la symptomatique négative, ainsi qu'un ou plusieurs inhibiteurs d'enzymes, outre des additifs et adjuvants pharmaceutiquement acceptables.

37. Préparation pharmaceutique selon la revendication 36, **caractérisée en ce qu'**il s'agit, pour l'inhibiteur d'enzymes ou les inhibiteurs d'enzymes d'un inhibiteur de la décarboxylase et/ou d'un inhibiteur de la catéchol-O-méthyltransférase et/ou d'un inhibiteur de la monoaminoxydase et/ou d'un inhibiteur de la β-hydroxylase.

38. Préparation pharmaceutique selon la revendication 37, **caractérisée en ce que** l'inhibiteur de la décarboxylase est choisi dans le groupe constitué par le D,L-sérine-2-(2,3,4-trihydroxybenzyl)-hydrazide (Benserazid), l'acide (-)-L-α-hydrazino-3,4-dihydroxy-α-méthylhydrocinnamique (Carbidopa), le L-sérine-2-(2,3,4-trihydroxybenzyl)hy**drazide, le glycine**-2-(2,3,4-trihydroxybenzyl)hydrazide et le L-tyrosine-2-(2,3,4-trihydroxybenzyl)hydrazide ainsi que leurs sels physiologiquement acceptables.

39. Préparation pharmaceutique selon la revendication 37, **caractérisée en ce que** l'inhibiteur de la catéchol-O-méthyltransférase est choisi parmi l'Entacapone et la Cabergoline ainsi que leurs sels physiologiquement acceptables.

40. Préparation pharmaceutique selon la revendication 37, **caractérisée en ce que** l'inhibiteur de la monoaminoxydase est choisi dans le groupe constitué par la Sélégiline, la Moclobémide et la Tranylcypromine ainsi que leurs sels physiologiquement acceptables.

41. Préparation pharmaceutique selon la revendication 37, **caractérisée en ce que** l'inhibiteur de la β-hydroxylase est choisi parmi le calcium-5-butylpicolinate et le calcium-5-pentylpicolinate ainsi que leurs sels physiologiquement acceptables.
